Europäisches Patentamt

European Patent Office

Office européen des brevets

(11) Publication number: 0 107 457

B1

# EUROPEAN PATENT SPECIFICATION

(45) Date of publication of patent specification: 26.02.86

(51) Int. Cl.⁴: $C\ 07\ C\ 85/06$, $C\ 07\ C\ 87/08$

(21) Application number: 83306236.7

(22) Date of filing: 14.10.83

(54) Manufacture of amines.

(30) Priority: 25.10.82 GB 8230464

(43) Date of publication of application:
02.05.84 Bulletin 84/18

(45) Publication of the grant of the patent:
26.02.86 Bulletin 86/09

(84) Designated Contracting States:
BE DE FR GB IT NL SE

(56) References cited:
EP-A-0 025 693
EP-A-0 040 016
GB-A-2 019 394
US-A-3 459 676
US-A-4 082 805

(73) Proprietor: IMPERIAL CHEMICAL INDUSTRIES
PLC
Imperial Chemical House Millbank
London SW1P 3JF (GB)

(72) Inventor: Tompsett, Alan John
11 Bransksome Grove
Hartburn Stockton-on-Tees Cleveland (GB)
Inventor: Whittam, Thomas Vincent
30 Wilton Drive
Darlington County Durham (GB)

(74) Representative: Martin, David Lincoln et al
Imperial Chemical Industries PLC Legal
Department: Patents PO Box 6 Bessemer Road
Welwyn Garden City Herts, AL7 1HD (GB)

Courier Press, Leamington Spa, England.

## Description

The present invention relates to the manufacture of methylamines.

Lower amines, for example, the three methylamines are prepared commonly by the reaction of ammonia with the corresponding alcohol, for example methanol. The reaction is usually effected in the vapour phase over a catalyst at a temperature in the range 300 to 500°C and at a pressure in the range 10 to 30 kg/cm². The catalyst which is most frequently used, especially for the manufacture of methylamines, is a dehydration catalyst, for example an oxide such as thoria, alumina, zirconia and silica, or, most usually, a mixed silica-alumina catalyst. It has also been proposed that various crystalline aluminosilicate zeolites be used as catalysts for the manufacture of amines. For example, U.S. Patent No. 4,082,805 describes the production of aliphatic amines over a catalyst comprising a crystalline aluminosilicate having the structure of ZSM-5, ZSM-11 or ZSM-21. UK Patent Application No. 2,019,394A describes a similar process using a catalyst comprising another zeolite, zeolite FU-1 which has been modified by replacement of some or all of the protons of the zeolite by monovalent cations, for example sodium. European Patent Application No. 25693 describes the production of monomethylamines in certain specified conditions using a catalyst selected from modified mordenite, modified ferrierite, erionite ore, calcium erionite and clinoptilolite ore.

In general in the manufacture of the three methylamines it is preferred to produce monomethylamine (MMA) and dimethylamine (DMA) in preference to trimethylamine (TMA). The greatest demand at the present time is for dimethylamine rather than monomethylamine and various proposals have been made to modify the amines manufacturing process so as to increase the yield of dimethylamine. Nevertheless there may be local situations where demand for monomethylamine predominates and, in any case, manufacturers and suppliers of amines must carefully tailor their output of all three amines to meet the specific demands of the market place. We have now surprisingly found that the amines process can be adapted to produce a good yield of MMA in preference to both DMA and TMA if a certain zeolite catalyst is used.

According to the present invention a process for the manufacture of methylamines comprises reacting a feed comprising methanol and/or dimethylether and ammonia over a catalyst comprising a synthetic levynite-type zeolite.

The zeolite levynite occurs naturally and levynite-type zeolites have also been synthesised. Examples of such synthetic zeolites are ZK-20, which is described in US Patent No. 3,459,676, and zeolite Nu-3. Zeolite Nu-3 is particularly preferred for use in the process of this invention and it is described and claimed in our European Patent Application published as EPA No. 40,016. Zeolite Nu-3 has a molar composition expressed by the formula

$$0.5 \text{ to } 1.5 \text{ } R_2O:Y_2O_3:\text{at least } 5 \text{ } XO_2:0 \text{ to } 400 \text{ } H_2O$$

wherein R is a monovalent cation or 1/n of a cation of valency n where n is a whole number of 2 or more, X is silicon and/or germanium, Y is one or more of aluminium, iron or gallium and $H_2O$ is water of hydration additional to water notionally present when R is H, and has an X-ray diffraction pattern (as determined by standard technique using copper Kα radiation) of which the principal lines are set out in Table 1. The X-ray pattern is affected in minor ways by the type of cation present.

TABLE 1
Typical X-ray data for zeolite Nu-3

| dA | I/Io | dA | I/Io |
|---|---|---|---|
| 10.1 ±0.2 | w→m | 3.12±0.05 | s |
| 8.0 ±0.14 | m→vs | 3.03±0.05 | w |
| 6.56±0.14 | w→vs | 2.98±0.05 | w→m |
| 5.54±0.10 | w | 2.81±0.05 | w |
| 5.09±0.10 | m→vs | 2.75±0.05 | m→s |
| 4.97±0.09 | w | 2.59±0.04 | w |
| 4.21±0.08 | s→vs | 2.50±0.03 | w |
| 4.02±0.07 | vs | 2.07±0.03 | w |
| 3.95±0.07 | w | 2.01±0.03 | w |
| 3.80±0.07 | m→vs | 1.90±0.02 | w |
| 3.77±0.06 | w→m | 1.86±0.02 | w |
| 3.66±0.06 | w→m | | |
| 3.54±0.06 | w | | |
| 3.42±0.06 | w→m | | |
| 3.28±0.05 | w→m | | |
| 3.18±0.05 | w | | |

vs=very strong with I/Io in the range 60 to 100
s=strong with I/Io in the range 40 to 60
m=medium with I/Io in the range 20 to 40
w=weak with I/Io in the range 0 to 20

Within the above definition of chemical composition the number of moles of $XO_2$ is typically in the range 5 to 1000 and zeolite Nu-3 appears to be most readily formed in a state of high purity when the number of moles of $XO_2$ is in the range 10 to 300, and the number of moles of $R_2$ is in the range 0.8 to 1.5.

This definition includes both freshly prepared zeolite Nu-3 ("freshly prepared" means the product of synthesis and washing, with optional drying as hereinafter described) and also forms of it resulting from dehydration, and/or calcination, and/or ion exchange. In freshly prepared zeolite Nu-3, R may include an alkali metal cation, especially sodium, and/or ammonium and usually includes nitrogen-containing organic cations such as derivatives of quinuclidine or cationic degradation products thereof, or precursors thereof, or mixtures of such nitrogen containing compounds. These nitrogen containing cations are hereinafter referred to as Q. Thus a zeolite Nu-3 as freshly made typically has the following molar composition:

0.3 to 1.2 $M_2O$:0.4 to 20 Q:$Y_2O_3$:5 to 1000 $XO_2$:0 to 400 $H_2O$

where M is an alkali metal or ammonium.

Table 2 shows X-ray data for zeolite Nu-3 as freshly prepared in the sodium N-methylquinuclidinium form and Table 3 shows X-ray data for zeolite Nu-3 in calcined sodium hydrogen form.

3

TABLE 2
Zeolite Nu-3 as freshly prepared in sodium
N-methylquinuclidinium form

| d(A) | I/Io |
|------|------|
| 10.11 | 8 |
| 8.01 | 33 |
| 6.56 | 19 |
| 5.50 | 10 |
| 5.07 | 79 |
| 4.94 | 14 |
| 4.62 | 2 |
| 4.21 | 56 |
| 4.01 | 100 |
| 3.78 | 35 |
| 3.54 | 6 |
| 3.42 | 3 |
| 3.27 | 18 |
| 3.18 | 2 |
| 3.12 | 48 |
| 3.03 | 9 |
| 2.81 | 5 |
| 2.75 | 38 |

### TABLE 3
#### Zeolite Nu-3 in calcined Na-H form

| d(A) | I/Io |
|------|------|
| 10.10 | 21 |
| 8.03 | 100 |
| 7.55 | 8 |
| 6.58 | 75 |
| 5.51 | 3 |
| 5.07 | 40 |
| 4.94 | 2 |
| 4.62 | 1 |
| 4.21 | 49 |
| 4.01 | 85 |
| 3.78 | 22 |
| 3.54 | 11 |
| 3.42 | 6 |
| 3.28 | 22 |
| 3.12 | 51 |
| 3.03 | 11 |
| 2.81 | 8 |
| 2.75 | 42 |

The $H_2O$ content of freshly prepared zeolite Nu-3 depends on the conditions in which it has been dried after synthesis.

In calcined forms of zeolite Nu-3, R may be alkali metal but includes less or no nitrogen-containing organic compounds, since these are burnt out in the presence of air or ammonia, leaving hydrogen as the other balancing cation.

Among the ion-exchanged forms of zeolite forms of zeolite Nu-3 the ammonium form is of importance since it can be readily converted to the hydrogen form by calcination. The hydrogen form can also be prepared directly by exchange with an acid.

Zeolite Nu-3 is prepared by a method which comprises reacting an aqueous mixture comprising at least one oxide $XO_2$, at least one oxide $Y_2O_3$ and at least one optionally substituted quinuclidinium ion.

Preferably the mixture has the molar composition:

$XO_2/Y_2O_3$ at least 5, preferably 10 to 300, especially 15 to 70;
$M^+/Q^+$ 0 to 2.0, preferably 0.2 to 0.8;
$H_2O/QZ$ 15 to 300, preferably 25 to 80;
$H_2O/XO_2$ 8 to 30, preferably 10 to 20;
$OH^-/XO_2$ 0.01 to 2.0, preferably 0.2 to 0.5;

where X is silicon and/or germanium, Y is one or more of aluminium, iron, or gallium, $M^+$ is an alkali metal ion, or mixtures of such ions which can include ammonium, and refers to free alkali, $Q^+$ is the aforesaid quinuclidinium ion or a precursor thereof, Z is hydroxide or is any acid radical capable of forming salts with the quinuclidinium ion, for example chloride, bromide or iodine and $OH^-$ refers to total free alkali metal, ammonium or any quaternary quinuclidinium compound or any combination of these. Whenever Z is an

5

acid radical, at least an equivalent of free alkali must be employed to maintain the desired level of neutrality or alkalinity.

Suitable quinuclidinium ions include the quinuclidinium ion itself and substituted quinuclidinium ions such as N-substituted derivatives (e.g. N-alkyl or N-hydroxyalkyl, preferably containing 1 to 6 carbon atoms) and/or substituted derivatives (including, for example, one or more alkyl and/or hydroxyalkyl groups, preferably containing from 1 to 6 carbon atoms). The preferred quinuclidinium ion is the N-methyl quinuclidinium ion.

The preparation and characteristics of zeolite Nu-3 are more fully described in our European Patent Application No. 40,016.

In the process of this invention it is preferred that the catalyst comprises binderless zeolite Nu-3. However the zeolite may be formed, if desired, into pellets in association with an inorganic matrix which can be either inert or catalytically active. Suitable inorganic materials include one or more of alumina, silica, kaolin clays, bentonites, montmorillonites, sepiolite, attapulgite, Fuller's earth and synthetic porous materials such as silica-alumina, silica-zirconia, silica-thoria, silica-beryllia, and silica-titania. Alternatively the zeolite may be used in the pure form (or with appropriate granulation aids) in the form of granules. Binders of the types hereinbefore listed may also be added to such granules to moderate or modify the catalytic activity.

It is preferred that the zeolite Nu-3 be in the hydrogen form prepared by ion-exchanging the zeolite using dilute hydrochloric or some other suitable acid, if required in the presence of a buffer.

It is further preferred to operate the amination process at a temperature in the range 300 to 450°C, more preferably in the range 350 to 400°C.

The alcohol liquid hourly space velocity used in the process of this invention is most suitably in the range 0.2 to 0.75 hr$^{-1}$. The degree of conversion of methanol will generally be within the range 60 to 98%, or more usually in the range 75 to 95%. However, these ranges are merely indicative and the process can be operated at degrees of conversion outside these ranges.

The N/R molar ratio, defined as the ratio of

$$\frac{\text{moles of ammonia} + \text{ammonia equivalent of recycled amine}}{\text{moles of alkyl (alcohol} + \text{recycled amine)}}$$

of the feed to the reactor is of some importance and is preferably in the range 0.5 to 5.0, more preferably in the range 1.0 to 2.5. The ratio chosen will affect the product distribution of mono-: di-: tri-methylamine, thus permitting the process operator some flexibility in operation. Nevertheless to exploit the invention most advantageously the reaction conditions will generally be chosen to use as low a N/R ratio as possible, consistent with obtaining a good yield of MMA, to minimise the recycle of excess ammonia. N/R molar ratios lower than 1.0 and more particularly below 0.5 may be used but can be expected to lead to side reactions and coke formation on the catalyst.

The process of this invention can conveniently form the first stage of a two-stage process for the production of dimethylamine. The second stage, in which the monomethylamine/rich product of the present process is converted, can utilise, for example, one or other of the processes described in our UK Patent 2,013,660B (in which the catalyst comprises zeolite FU-1) and our European Patent Application published as No. 76034 in which the preferred catalyst comprises a binderless zeolite 5A.

The process of this invention is further illustrated in the following Examples.

Example 1

A laboratory microreactor was charged with a bed (6 ml) of unsupported zeolite sodium hydrogen Nu-3 in the form of particles of 12 to 24 mesh B.S.S. The zeolite had been obtained by calcination and partial HCl exchange of zeolite Nu-3 of composition 0.96 Na$_2$O:Al$_2$O$_3$:21 SiO$_2$. In this form it had a moderate hydrogen ion content and composition 0.3 Na$_2$O:Al$_2$O$_3$:22 SiO$_2$. It was conditioned overnight by treatment with a stream of nitrogen at 360°C. A feed of methanol (40% molar) and ammonia (60% molar) was then passed over the catalyst at a liquid hourly space velocity of 0.63 hr$^{-1}$ and an N/R molar ratio of 1.5. The initial temperature was 360°C and this was increased during the run in two steps of 20°C to a final temperature of 400°C. Details of the run and of the products obtained are given in Table 4.

Example 2

A similar microreactor to that used in Example 1 was charged with 5.5 ml (16 to 25 mesh B.S.S.) of a low sodium form of H-Nu-3 zeolite derived by calcination and HCl exchange of freshly made zeolite Nu-3 of composition 0.42 Na$_2$O:Al$_2$O$_3$:60 SiO$_2$. The zeolite used analysed as 0.002 Na$_2$O:Al$_2$O$_3$:61 SiO$_2$, the balance of the framework charge being protons. It was conditioned overnight, as in Example 1, and a feed of methanol and ammonia was passed over it under conditions comparable to those used in Example 1. Details of the run and of the products obtained are given in Table 5.

TABLE 4

| Run No. | Temperature °C | Product analysis mole % | | | | | | % Weight conversion | | | Amines analysis | | | | | |
|---|---|---|---|---|---|---|---|---|---|---|---|---|---|---|---|---|
| | | NH₃ | CH₄ | M | D | T | MeOH | To amines | To by-prod | Total | Mole % | | | Weight % | | |
| | | | | | | | | | | | M | D | T | M | D | T |
| 1 | 360 | 27.1 | 0 | 27.4 | 3.1 | 0.1 | 4.9 | 83.9 | 3.8* | 87.8 | 89.6 | 10.0 | 0.4 | 85.4 | 13.8 | 0.8 |
| 2 | 360 | 28.3 | 0 | 27.9 | 2.7 | 0.1 | 5.2 | 83.6 | 2.5* | 87.1 | 90.9 | 8.7 | 0.4 | 87.2 | 12.1 | 0.7 |
| 3 | 380 | 25.0 | 0 | 30.2 | 2.8 | 0.2 | 2.4 | 90.5 | 3.6* | 94.1 | 91.1 | 8.4 | 0.5 | 87.3 | 11.7 | 0.9 |
| 4 | 400 | 24.7 | 0.1 | 29.3 | 3.5 | 0.3 | 1.2 | 93.0 | 3.9* | 96.9 | 88.5 | 10.6 | 0.9 | 83.8 | 14.5 | 1.7 |

M=Monomethylamine
D=Dimethylamine
T=Trimethylamine
*=Predominantly dimethyl ether

TABLE 5

| Run No. | Temperature °C | Product analysis mole % | | | | | | % Weight conversion | | | Amines analysis | | |
|---|---|---|---|---|---|---|---|---|---|---|---|---|---|
| | | NH₃ | CH₄ | M | D | T | MeOH | To amines | To by-prod | Total | Mole % | | |
| | | | | | | | | | | | M | D | T |
| 1 | 360 | 36.6 | 0 | 12.4 | 7.7 | 5.3 | 0.2 | 95.6 | 3.9* | 99.5 | 48.7 | 30.0 | 21.0 |
| 2 | 380 | 40.4 | 0.1+ | 8.8 | 6.5 | 7.0 | 0.2 | 96.6 | 3.0* | 99.6 | 39.6 | 29.1 | 31.2 |
| 3 | 400 | 42.7 | 0.2+ | 8.7 | 5.8 | 6.6 | 0.2 | 96.6 | 2.8* | 99.5 | 41.1 | 27.7 | 31.2 |

M=Monomethylamine
D=Dimethylamine
T=Trimethylamine
*=Predominantly dimethyl ether
+=Plus 0.1 mole % ethane

These results indicate that zeolite Nu-3 is not only a useful catalyst for the manufacture of methylamines but also in its various forms is selective for production either of MMA in strong preference to DMA and TMA, or of MMA and DMA in preference to TMA. These results may usefully be contrasted with those from an unselective catalyst, such as an amorphous silica-alumina of the type currently widely used in methylamines production processes, shown in Table 6. The catalyst was charged as washed sieved particles of 16 to 25 mesh B.S.S. The amine product ratio from this catalyst is close to the thermodynamic equilibrium level, except when the process is operated at low conversion.

Without wishing to be limited by any theory we believe the selectivity behaviour differences between the different samples of Nu-3 may be associated with the nature and ratio of exchangeable cations, but variations in the $SiO_2/Al_2O_3$ ratio may also make a contribution.

TABLE 6

| Run No. | Temp. °C | Methanol LHSV hr | Feed N/R molar | Product analysis mole % | | | | | | % Weight conversion | | | Amines analysis | | | | | |
|---|---|---|---|---|---|---|---|---|---|---|---|---|---|---|---|---|---|---|
| | | | | NH$_3$ | CH$_4$ | M | D | ·T | MeOH | To amines | To by-prod* | Total | Mole % | | | Weight % | | |
| | | | | | | | | | | | | | M | D | T | M | D | T |
| 1 | 360 | .63 | 1.5 | 40.9 | 0 | 6.1 | 4.3 | 7.1 | 2.0 | 92.5 | 2.5 | 95.0 | 34.7 | 24.5 | 40.8 | 23.5 | 24.0 | 52.5 |
| 1A | 360 | .63 | 1.5 | 42.0 | 0 | 6.0 | 4.2 | 6.9 | 2.4 | 91.2 | 2.4 | 93.7 | 35.3 | 24.3 | 40.3 | 24.0 | 24.0 | 52.1 |
| 1B | 360 | .63 | 1.5 | 40.6 | 0 | 5.8 | 4.1 | 7.0 | 3.0 | 89.6 | 2.6 | 92.2 | 34.4 | 24.1 | 41.5 | 23.2 | 23.6 | 53.2 |
| 2 | 360 | .31 | 2.0 | 48.9 | 0 | 6.2 | 4.0 | 5.8 | 0.2 | 98.1 | 1.39 | 99.5 | 38.6 | 25.3 | 36.1 | 26.8 | 25.5 | 47.7 |
| 2A | 360 | .31 | 2.0 | 48.0 | 0 | 6.3 | 4.1 | 5.9 | 0.2 | 97.8 | 1.55 | 99.4 | 38.9 | 25.1 | 36.0 | 27.1 | 25.3 | 47.6 |
| 3 | 340 | .31 | 2.0 | 52.0 | 0 | 5.5 | 2.9 | 4.9 | 5.1 | 81.9 | 2.0 | 83.9 | 41.4 | 21.8 | 36.8 | 28.9 | 22.1 | 48.9 |
| 4 | 320 | .31 | 2.0 | 55.4 | 0 | 4.8 | 1.8 | 3.1 | 13.5 | 55.7 | 1.6 | 57.3 | 49.6 | 18.9 | 31.5 | 36.2 | 20.0 | 43.8 |

*=largely dimethyl ether

0 107 457

9

**Claims**

1. A process for the manufacture of methylamines which comprises reacting a feed comprising methanol and/or dimethylether and ammonia over a catalyst comprising a synthetic levynite-type zeolite.

2. A process as claimed in claim 1 in which the catalyst comprises zeolite Nu-3 having a molar composition expressed by the formula

$$0.5 \text{ to } 1.5 \text{ } R_2O:Y_2O_3:\text{at least } 5 \text{ } XO_2:0 \text{ to } 400 \text{ } H_2O$$

wherein R is a monovalent cation or 1/n of a cation of valency n where n is a whole number of 2 or more, X is silicon and/or germanium, Y is one or more of aluminium, iron or gallium and $H_2O$ is water of hydration additional to water notionally present when R is H, and has an X-ray diffraction pattern (as determined by standard technique using copper $K\alpha$ radiation) of which the principal lines are set out in Table 1.

3. A process as claimed in claim 1 in which the catalyst comprises freshly prepared zeolite Nu-3 having a molar composition expressed by the formula:

$$0.3 \text{ to } 1.2 \text{ } M_2O:0.4 \text{ to } 20 \text{ } Q:Y_2O_3:5 \text{ to } 1000 \text{ } XO_2:0 \text{ to } 400 \text{ } H_2O$$

where M is an alkali metal or ammonium, Q is a nitrogen-containing organic cation and having an X-ray diffraction pattern substantially as set out in Table 2.

4. A process as claimed in any one of the preceding claims in which the catalyst comprises binderless zeolite Nu-3.

5. A process as claimed in any one of the preceding claims in which the zeolite comprises zeolite Nu-3 in either the sodium/hydrogen form or the hydrogen (low sodium) form.

6. A process as claimed in any one of the preceding claims in which the amination process is operated at a temperature in the range 300 to 450°C and at an alcohol liquid hourly space velocity in the range 0.2 to 0.75 $hr^{-1}$.

7. A process as claimed in any one of the preceding claims in which the amination process is operated at an N/R molar ratio as hereinbefore defined, in the range 0.5 to 5.0.

8. A process as claimed in any one of the preceding claims in which at least some of the monomethylamine produced is converted in a further process stage to dimethylamine by reaction over a zeolite catalyst comprising zeolite Fu-1 or binderless zeolite 5A.

**Patentansprüche**

1. Verfahren zur Herstellung von Methylaminen, bei dem ein Einsatzmaterial, das Methanol und/oder Dimethylether und Ammoniak enthält, über einem Katalysator, der aus einem synthetischen Zeolith des Levynittyps besteht, zur Reaktion gebracht wird.

2. Verfahren nach Anspruch 1, bei dem der Katalysator aus Zeolith Nu-3 mit einer molaren Zusammensetzung, die durch die Formel:

$$0,5 \text{ bis } 1,5 \text{ } R_2O:Y_2O_3:\text{mindestens } 5 \text{ } XO_2:0 \text{ bis } 400 \text{ } H_2O$$

ausgedrückt wird, worin R ein einwertiges Kation oder 1/n eines Kations mit der Wertigkeit n ist, wobei n eine ganze Zahl ist, die 2 oder mehr als 2 beträgt, X Silicium und/oder Germanium ist, Y ein oder mehr als ein Element ist, das aus Aluminium, Eisen und Gallium ausgewählt ist, und $H_2O$ Hydratwasser ist, das zu Wasser hinzukommt, das begrifflich vorhanden ist, wenn R H ist, besteht und ein (durch ein Standardverfahren unter Anwendung von Kupfer-$K\alpha$-Strahlung ermitteltes) Röntgenbeugungsbild liefert, dessen Hauptlinien in Tabelle 1 dargelegt sind.

3. Verfahren nach Anspruch 1, bei dem der Katalysator aus frisch hergestelltem Zeolith Nu-3 besteht, der eine molare Zusammensetzung hat, die durch die Formel:

$$0,3 \text{ bis } 1,2 \text{ } M_2O:0,4 \text{ bis } 20 \text{ } Q:Y_2O_3:5 \text{ bis } 1000 \text{ } XO_2:0 \text{ bis } 400 \text{ } H_2O$$

ausgedrückt wird, worin M ein Alkalimetall oder Ammonium ist und Q ein stickstoffhaltiges organisches Kation ist, und im wesentlichen ein Röntgenbeugungsbild liefert, wie es in Tabelle 2 dargelegt ist.

4. Verfahren nach einem der vorhergehenden Ansprüche, bei dem der Katalysator aus bindemittelfreiem Zeolith Nu-3 besteht.

5. Verfahren nach einem der vorhergehenden Ansprüche, bei dem der Zeolith aus Zeolith Nu-3 in entweder der Natrium/Wasserstoff-Form oder der (einen niedrigen Natriumgehalt aufweisenden) Wasserstofform besteht.

6. Verfahren nach einem der vorhergehenden Ansprüche, bei dem das Aminierungsverfahren bei einer Temperatur von 300 bis 450°C mit einer in dem Bereich von 0,2 bis 0,75 $h^{-1}$ liegenden stündlichen Flüssigkeits-Raumgeschwindigkeit des Alkohols durchgeführt wird.

7. Verfahren nach einem der vorhergehenden Ansprüche, bei dem das Aminierungsverfahren mit

**0 107 457**

einem in dem Bereich von 0,5 bis 5,0 liegenden N/R-Molverhältnis, wie es vorstehend definiert ist, durchgeführt wird.

8. Verfahren nach einem der vorhergehenden Ansprüche, bei dem mindestens etwas von dem erzeugten Monomethylamin in einer weiteren Verfahrensstufe durch Umsetzung über einem Zeolith-Katalysator, der aus Zeolith FU-1 oder aus bindemittelfreiem Zeolith 5A besteht, in Dimethylamin ungewandelt wird.

**Revendications**

1. Procédé de production de méthylamines, qui comprend la réaction d'une alimentation comprenant du méthanol et/ou de l'éther diméthylique et de l'ammoniac sur un catalyseur comprenant une zéolite synthétique du type lévynite.

2. Procédé suivant la revendication 1, dans lequel le catalyseur comprend de la zéolite Nu-3 ayant une composition molaire exprimée par la formule:

$$\text{0,5 à 1,5 } R_2O:Y_2O_3:\text{au moins 5 } XO_2:\text{0 à 400 } H_2O$$

où R représente un cation monovalent ou 1/n d'un cation de valence n, où n représente un nombre entier de 2 ou davantage, X représente le silicium et/ou le germanium, Y représente l'un ou plusieurs d'entre l'aluminium, le fer et le gallium et $H_2$ représente l'eau d'hydratation en plus de l'eau présente en principe lorsque R représente H, et présentent un diagramme de diffraction des rayons X (tel que déterminé suivant la technique habituelle au moyen du rayonnement Kα du cuivre) dont les raies principales sont indiquées au tableau I.

3. Procédé suivant la revendication 1, dans lequel le catalyseur comprend de la zéolite Nu-3 fraîchement préparée ayant une composition molaire exprimée par la formule:

$$\text{0,3 à 1,2 } M_2O:\text{ 0,4 à 20 } Q:Y_2O_3:\text{5 à 1000 } XO_2:\text{0 à 400 } H_2O$$

où M représente un métal alcalin ou l'ammonium, Q représente un cation organique contenant de l'azote, et ayant un diagramme de diffraction des rayons X en substance tel qu'indiqué au tableau II.

4. Procédé suivant l'une quelconque des revendications précédentes, dans lequel le catalyseur comprend de la zéolite Nu-3 exempte de liant.

5. Procédé suivant l'une quelconque des revendications précédentes, dans lequel la zéolite comprend de la zéolite Nu-3 sous la forme sodium-hydrogène ou sous la forme hydrogène (pauvre en sodium).

6. Procédé suivant l'une quelconque des revendications précédentes, dans lequel le procédé d'amination est exécuté à une température de l'intervalle de 300 à 450°C et à une vitesse spatiale horaire liquide de l'alcool de l'intervalle de 0,2 à 0,75 $h^{-1}$.

7. Procédé suivant l'une quelconque des revendications précédentes, dans lequel le procédé d'amination est exécuté à un rapport molaire N/R tel que défini ci-dessus de l'intervalle de 0,5 à 5,0.

8. Procédé suivant l'une quelconque des revendications précédentes, dans lequel au moins une certaine quantité de la monométhylamine produite est convertie à un autre stade du procédé en diméthylamine par réaction sur une zéolite catalytique comprenant de la zéolite FU-1 ou de la zéolite 5A exempte de liant.

11